# EUROPEAN PATENT APPLICATION

(11) **EP 2 497 468 A1**
(43) Date of publication of application: **12.09.2012**
(21) Application number: 11157874.6
(22) Date of filing: 11.03.2011
(51) Int. Cl.: A61K 31/16, A61P 19/02, A61L 27/16, A61L 27/52, C08F 220/56, C08L 33/26

(54) **Polyacrylamide hydrogel for use in the treatment and/or prevention of joint swelling and/or bone oedema in a mammal suffering from arthritis**

(71) Applicant: Contura A/S, 2860 Søborg (DK)
(72) Inventor: Ankorina-Stark, Ieva, 2990 Nivå (DK)
(74) Representative: Plougmann & Vingtoft A/S

(57) **Abstract**

The present invention relates to a hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution for use in the treatment and/or prevention of joint swelling and/or bone oedema in a mammal suffering from arthritis such as osteoarthritis. Preferably, the hydrogel consists of 97.5% apyrogenic water and 2.5% polyacrylamide. The administration of 0.25 ml to 10 ml of the hydrogel is performed by intraarticular injection under sterile conditions.

The mammal suffering from arthritis is a preferably a human, a racing animal or a companion animal. If the mammal is a human, the joint or joints which is/are to be injected include the knee, hip, elbow, the metacarpal-phalangeal and interphalangeal joints in hands and feet, the sesamoid joint and/or the temperomandibular joint. If the mammal to be treated is a horse, the joint or joints which is/are to be injected include the fetlock, coffin, pastern, stifle, and/or knee joint of the hind legs. If the mammal to be treated is a dog, the joint or joints which is/are to be injected include the elbow of the front leg or the knee or hip joint of the hind legs.

## Description

### FIELD OF INVENTION

The present invention relates to a hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution for use in the treatment and/or prevention of joint swelling and/or bone oedema in a mammal suffering from arthritis.

### BACKGROUND OF THE INVENTION

Arthritis is a group of conditions involving damage to the joints of the body. There are over 100 different forms of arthritis. The most common form, osteoarthritis (degenerative joint disease) is a result of trauma to the joint, infection of the joint, or age. Other arthritis forms are rheumatoid arthritis, psoriatic arthritis, and related autoimmune diseases. Septic arthritis is caused by joint infection.

Regardless of the type of arthritis, the common symptoms for all arthritis disorders include varied levels of pain, swelling, joint stiffness and sometimes a constant ache around the joint(s). The major complaint by individuals who have arthritis is joint pain. Pain is often a constant and may be localized to the joint affected. The pain from arthritis occurs due to inflammation that occurs around the joint, damage to the joint from disease, daily wear and tear of joint, muscle strains caused by forceful movements against stiff, painful joints and fatigue.

Osteoarthritis (OA) is a painful, debilitating joint disease with no known cure. It is characterized by heat, pain, swelling, crepitus (a crackling, crinkly, or grating feeling or sound under the skin), and a decreased range of motion in affected joints. In humans it affects the hands, knees, hips, spine and other joints. Horses suffer from joint osteoarthritis in primarily coffin, pastern, fetlock, carpal and stiffle joints, and the incidence is dependent on age, weight and breed.

In some light cases rest and use of ice packs may be all that is needed to reduce inflammation and swelling, but in established cases treatment includes analgesic (pain-killing) and anti-inflammatory medications such as non-steroid anti-inflammatory drugs and steroid, exercise management and, occasionally, surgery.

Visco-supplementation is the process of injecting a gel-like substance into the joint. The substance is thought of as an additive to the joint fluid, thus lubricating the cartilage, reducing pain and improved joint flexibility. This method of treatment, however, requires ongoing injections, as benefits are only temporary, because the currently used substances are degradable within weeks to months. Substances used in visco-supplementation include hyaluronic acid, or HA (Legend®, Hylartin® and Synacid®, Synvisc, Euflexxa, Supartz etc) and poly-sulfated glycosaminoglycans (PSGAGS) such as Adequan®.

### SUMMARY OF THE INVENTION

The present invention relates to a hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution for use in the treatment and/or prevention of joint swelling and/or bone oedema in a mammal suffering from arthritis.

The hydrogel typically has a molecular weight between 0.01 x 10⁶ and 20 x 10⁶. The polymer is resistant to biological degradation. The polyacrylamide hydrogel of the invention is fully biocompatible (according to ISO standard test ISO-10993). The polyacrylamide hydrogel does not have cytotoxic effect on human fibroblasts, is non-toxic, non-carcinogenic, non-allergenic, non-mutagenic, and resistant to enzymatic and microbiological degradation. Furthermore, the polymer is not water-soluble.

However, chemical substances can pass and cells enter the polymer which can act as a three-dimensial matrix to provide structure for the cells in a similar manner as Matrigel™.

Polyacrylamide hydrogel (PAAG) is a non-particulate homogenous gel consisting of e.g. 97.5% apyrogenic water and 2.5% polyacrylamide (polymer). It is similar to hyaluronic acid gel in chemical composition and tissue compatibility but with a longer-lasting effect, as it is non-degradable. This gel has been used for years in the augmentation of connective tissue, and experimental studies supported by histopathological observations have shown that it exerts its effect by being integrated within the tissue through a combination of vessel in-growth and molecular water exchange. Vessel in-growth begins with host macrophages and foreign-body giant cells entering the gel being gradually replaced by fibroblasts and endothelial cells, which form a thin vessel-bearing fibrous network.

It has surprisingly been found that PAAG is also useful in the treatment or prevention of joint swelling and bone oedema in a mammal suffering from arthritis.

### DETAILED DESCRIPTION OF THE INVENTION

The hydrogel is prepared as described in WO 02/16453, hereby incorporated by reference.

The hydrogel may comprise any embodiment of the hydrogel as described in WO 02/16453. Preferably, the hydrogel comprises 0.5 to 25% by weight polyacrylamide, based on the total weight of the hydrogel. The hydrogel typically further comprises at least 75% by weight pyrogen-free water or saline solution, preferably pyrogen-free water. It may be administered by implantation or injection into the intra-articular cavity of a joint.

The hydrogel is obtainable by combining acrylamide and methylene bis-acrylamide monomers, initiating polymerisation by radical initiation; and washing with pyrogen-free water or saline solution, the combining being in amounts and the washing being such as to give about 0.5 to 25% by weight polyacrylamide, based on the total weight of the hydrogel. The hydrogel thus obtained is both biostable and biocompatible, and is not resorbed by the body. Moreover, the hydrogel is resilient to mechanical stress.

Typically, the hydrogel is obtained by combining acrylamide and methylene bis-acrylamide is in a molar ratio of 150:1 to 1000:1. Methylene bis-acrylamide serves to provide cross-linking between polymer chains and the molar ratio may be varied to provide various cross-linking densities of the hydrogel. The conditions for obtaining the hydrogel may be modified according to, for instance, the nature of the joint into which the hydrogel is intended to be injected. The desired rheological properties, such as elasticity, may be controlled at least in part by the solid weight content of the hydrogel. The hydrogel of the invention comprises about 0.5 to 25% by weight polyacrylamide, based on the total weight of the hydrogel. In suitable embodiments of the invention, the hydrogel comprises less than 15% by weight polyacrylamide, based on the total weight of the hydrogel, preferably less than 10% by weight, more preferably less than 7.5% by weight, even more preferably less than 5%, most preferably less than 3.5% by weight polyacrylamide, based on the total weight of the hydrogel.

The combining involves the combining of the component reagents acrylamide and methylene bis-acrylamide, typically degassed and typically in a manner to minimise operator contact. The reagent components may optionally be combined previously to form an inert mixture. An inert mixture is one wherein no chemical reaction proceeds among the component reagents. The combining involves combining acrylamide, methylene-bis-acrylamide, and a radical initiator component to initiate polymerisation. In a suitable embodiment, an inert premixture of acrylamide, methylene-bis-acrylamide (the cross-linker) and N,N,N',N'-tetramethylene-ethylene-diamine (TEMED) is combined with an ammonium persulfate (AMPS) initiator solution. However, the components may be combined as singularities or as alternative plural premixtures.

Acrylamide and methylene-bis-acrylamide are suitably combined in a molar ratio of about 150:1 to 1000:1, typically about 150:1 to 900:1, preferably about 175:1 to 800:1, more preferably about 200:1 to 600:1, most preferably from 250:1 to 600:1. As shown in Tables 2 and 3, hydrogels of differing solid-weight content and rheological properties may be controllably prepared. The hydrogel having the desired rheological characteristics has been obtained by combining acrylamide and methylene-bis-acrylamide in a ratio of about 250:1, about 260:1, about 270:1, about 280:1, about 290:1, about about 300:1, about 310:1, about 320:1, about 330:1, about 340:1, about 350:1, about 360:1, about 370:1, about 380:1, about 390:1, about 400:1, about 410:1, about 420:1, about 430:1, about 440:1, about 450:1, about 460:1, about 470:1, about 480:1, about 490:1 and about 500:1.

Particularly in the embodiment wherein the hydrogel is injected into a joint, the elasticity of the hydrogel is of great relevance. In a preferred embodiment, the hydrogel of the invention has an elasticity modulus of about 1 to 200 Pa, such as about 2 to 175 Pa, typically about 5 to 150 Pa, such as 10 to 100 Pa.

The hydrogel comprises at least 75% by weight pyrogen-free water or saline solution, preferably pyrogen-free water. In a suitable embodiment of the invention, the hydrogel comprises at least 80% by weight pyrogen-free water or saline solution, preferably at least 85 %, more preferably at least 90%, even more preferably at least 95% by weight pyrogen-free water or saline solution.

A suitable saline solution has an osmolarity similar to that of interstitial fluid. Suitable saline solutions include but are not limited to the group selected from 0.25- 1% aqueous sodium chloride, a Ringer-Lockart solution, an Earle solution, a Hanks solution, an Eagle medium, a 0.25 - 1% glucose solution, a potassium chloride solution, and a calcium chloride solution. In a preferred embodiment, the saline solution is a 0.8-1 % aqueous sodium chloride solution, such as a 0.8, 0.9 or 1 % aqueous sodium chloride solution, most preferably about 0.9 % aqueous sodium chloride.

As will be obvious to the person skilled in the art, in the embodiment wherein saline solution is used either for the preparation of the gel and/or for the washing of the gel, the solid-weight content of the gel will be higher than the contribution made by the polyacrylamide, but typically not more than an additional 1%.

In a particularly suitable embodiment of the invention, the hydrogel comprises about 2.5 % by weight polyacrylamide, based on the total weight of the hydrogel and about 97.5 % pyrogen-free water.

Pyrogen-free water or saline solution is used for the washing process. The washing process serves, in part, to remove all but trace amounts of the monomers acrylamide and N,N'-methylene-bis-acrylamide. These monomers are toxic to the patient as well as detrimental to the stability of the hydrogel. The washing process is preferably such that the concentrations of the remaining monomers acrylamide and N,N'-methylene-bis-acrylamide are below 50 ppm, more preferably below 40 ppm, such as below 30 ppm, most preferably below 20 ppm, typically below 10 ppm, particularly preferably below 5 ppm.

It has been found that a hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution is useful in the treatment and/or prevention of joint swelling and/or bone oedema in a mammal suffering from arthritis.

A small amount of fluid exists in normal joints. Joint swelling can be a primary symptom of arthritis. When a joint is affected by arthritis, inflammatory types of arthritis in particular, increased abnormal amounts of fluid build up, making the joint swollen. The excess fluid is produced by the soft tissues affected by arthritis that surround and line the joints As used herein, "joint swelling" means the buildup of fluid in the soft tissue surrounding the joint. The swelling may cause the joint to appear larger or abnormally shaped.

The hydrogel is implanted or injected into the joint(s) which are to be treated, preferably by injection. The injection of the gel may be performed under local anaesthesia, but local anaesthesia is not necessarily required. However, the procedure must be performed under sterile conditions. Any hair covering the injection area is cropped and the skin thoroughly rinsed e.g. with chlorhexidine and ethanol (e.g. 3 times interchangeably). Then, the cannula is inserted into the joint cavity and it is checked by aspiration that it is placed properly intraarticularly. Generally, the joint is emptied for at least the amount of liquid which it has been decided to inject and the desired amount of the gel is then injected. An antibiotic may be included in the gel in order to prevent iatrogenic infection of the joint.

An appropriate amount of hydrogel will be in the range of 0.25 ml to 10 ml, such as 0.3, 0.4, 0.5, 0.6, 0.7, 0.8, 0.9, 1, 2, 3, 4, 5, 6, 7, 8 or 9 ml. The effect appears to improve with a larger amount. However, it is not advisable to inject so much hydrogel that the joint is expanded. The exact amount will be decided by the treating physician or veterinarian on the basis of the size of the joint and the severity of the symptoms.

One injection or a series of injections is followed by an evaluation of the degree of symptoms relief provided by the hydrogel or as objective improvement e.g. by MRI and followed by one or more injections if required. This procedure may be repeated as often as required to alleviate joint swelling and/or bone oedema. The treatment may be performed as a treatment of one or more affected joints or as prevention to avoid joint swelling and/or bone oedema, in particular but not limited to a joint which has previously been affected by joint swelling and/or bone oedema or a joint which is contralateral to a joint which has previously been affected by joint swelling and/or bone oedema.

The present invention is relevant for treatment and/or prevention of joint swelling and/or bone oedema in any mammal suffering from any form of arthritis.Most relevant mammals are humans, racing animals such as horses and camels, and companion animals such as cats and dogs but also other mammals in need of treatment such as elephants, giraffes, tigers etc.

Any joint affected by arthritis may be treated. Non-limiting examples are knee, hip, elbow, the metacarpal-phalangeal and interphalangeal joints in hands and feet of humans, fetlock, coffin, pastern, stiffel, and knee joint of the hind legs of horses, and elbow, knee and hip of cats and dogs. Also treatment of other joints such as the sesamoid and the temperomandibular joints may be relevant.

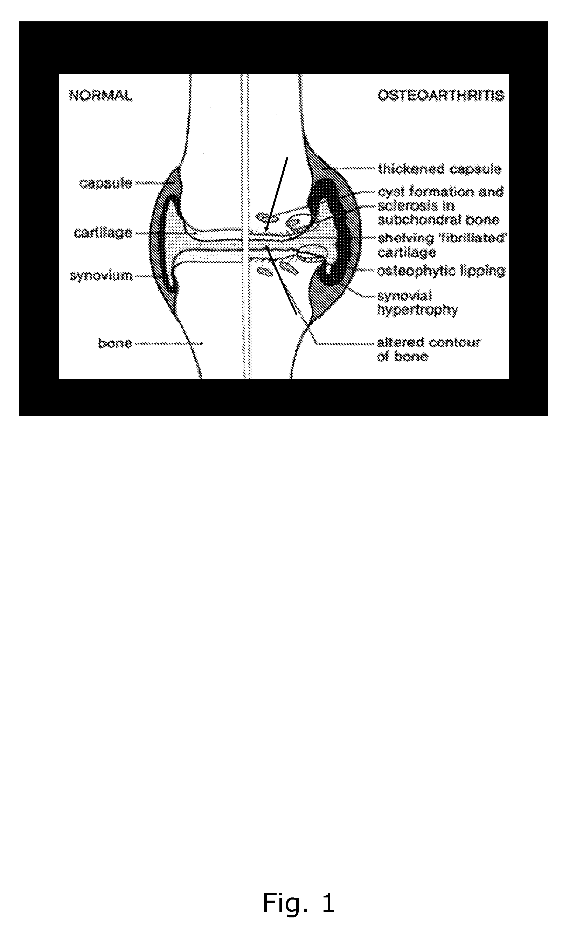

Osteoarthris is the most common form of arthritis. Characteristics of osteoarthritis include a loss of cartilage, seen as a reduction in joint space, and osteophytes (marginal lips of bone that grow at the edges of the joints) (see figure 1). However, other forms of arthritis are also characterized by joint space loss and the diffential diagnosis between different forms of arthritis may require specialist knowledge in particular as many of the symptoms such as joint swelling and pain are found in several forms of arthritis; the distinction between the various forms of arthritis is of minor relevance as the present invention will be relevant for most if not all of them.

As used herein, "osteoarthritis" means a non-inflammatory or minimally inflammatory arthritis in which there is degeneration of articular cartilage, thickening, erosion and subarticular cyst formation of the underlying subchondral bone, and productive of reactive marginal osteophytes about the joint, leading to deformity from joint destruction (see figure 1).

As used herein, "joint space loss" means a continued loss or increased abnormality of cartilage (commonly associated with joint space loss). Joint space loss can determined by radiography. Bone scintigraphy is useful in determining bone functional status. Magnetic Resonance Imaging (MRI) is a non-invasive cross sectional imaging technique that can demonstrate specific soft tissue changes, for examples the presence of fluid within the bone marrow. Increasingly MRIs are used to evaluate joints of patients with arthritis. The MRI is a very sensitive technique that allows better visualization of the bones, cartilage, and joint tissue. An MRI picture is in black and white like an XRay but can provide cross sectional pictures from several different perspectives. "Bone marrow oedema" can be seen in a number of different conditions. While the term "oedema" implies fluid in the area, there are actually many different conditions that can cause this MR1 finding. This can be seen in osteoarthritis in bone under areas where cartilage is damaged; in rheumatoid arthritis oedema may precede an erosion developing; in ankylosing spondylitis oedema may precede the development of tendon insertional calcification.

As used herein, "bone marrow oedema" means increased fluid content within the subchondral bone marrow above that in normal subchondral bone marrow.

Determination of the presence of bone marrow oedema about or of the joint is predictive of the subject developing progressive osteoarthritis in the joint (cf. US 6,564,083). As used herein, "progressive osteoarthritis" means a continued loss of cartilage (commonly with joint space loss) and bone stock, as well as the increase in marginal ostephytes, subchondral bone hardening/thickening, and the onset or increase of subchondral cysts, as well as other finding known to occur with osteoarthritis. Oedema is often seen in association with cysts, which are known to be associated with osteoclastic bone absorption.

The present invention provides a method of treating and/or preventing bone oedema in a mammal suffering from arthritis, the method comprising injecting intraarticularly under sterile conditions a hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution. It is contemplated that the administration will also prevent joint swelling and/or osteoclastic bone absorption.

In a preferred embodiment of the invention the mammal is suffering from osteoarthritis.

As demonstrated in the examples injection of PAAG into mammals with joints affected by swelling and/or bone oedema has successfully relieved the symptoms associated with the joint swelling and/or bone oedema i.e. halting. The mode of action appears to be through a buffer-like capacity of the synovial lining, as the gel becomes intimately integrated within the soft tissues herniating into the joint cavity. The gel is not being degraded, and it is possible that injections of the hydrogel may exert a cushioning effect on the joint by not only supporting and preserving the synovial lining but perhaps also by allowing regeneration of the underlying damaged joint cartilage,soft tissues and bone.

It is hypothesized by the present inventor that the effect may also be a result of scavenging of the calcium pyrophosphate dehydrate (CPPD) and basic calcium phosphate (BCP) crystals commonly deposited in osteoarthritic joint (Liu et al., 2009)

### LEGENDS TO FIGURES

Fig. 1. All the nerve receptors are situated within the bone, which is very close to the surface in osteoarthritis due to cartilage thinning (arrows) and lack of cartilage. Bone spurs (circles) may develop.
Fig. 2. The rabbit inner capsule lining after 6 months. The gel is localized beneath the synovial cell lining (arrows).
Fig. 3. Fetlock joint injected with gel 8 months previously. Gel was identified subsynovially macroscopically (arrows) as well as microscopically (see Fig 4). Note that the gel deposits were concentrated at sites, where transition (of the synovialis) occurred from connective tissue of the joint capsule to cartilage (arrows).
Fig. 4. PAAG gel lying within the soft tissue (arrows in upper figures) or as a cushion beneath the synovial cells (arrows in lower figures).

### EXAMPLES

### Example 1

### Preparation of Hydrogel:

The gel is a polyacrylamide gel manufactured by a polymerisation of the monomers of acrylamide (AM) and N,N'-methylene-bis-acrylamide (bisAM). The finished product may have different elasticity modules.

The hydrogel typically contains approximately 95% water. The concentration of the monomers acrylamide and N,N'-methylene-bis-acrylamide has been shown to be less than 10 ppm and is adequate for the desired stability of the final product, often less than 5 ppm.

The finished product must conform with respect to pH, absence of heavy metals, refractive index, stability, absence of pyrogens, and must be sterile, practically inert, and be substantially free of monomers.

### Preparation 1.1

The synthetic preparation suitably involves the following operations:
1. Two mixtures, A1 and A2, are prepared. A1 comprises water, acrylamide, N,N'-methylene-bis-acrylamide, N,N,N',N'-tetramethylene-ethylene-diamine (TEMED). A2 comprises water and ammonium persulfate;
2. The two mixtures are combined in the following ratio: 1990 mL of A1 and 10 mL of A2 and kept at 45 °C and degassed with nitrogen for 20 seconds;
3. The reaction mixture is cast into several 100 mL beakers;
4. Polymerisation is allowed to occur for 0.5 to 1.5 hours;
5. The gel is demolded;
6. Residual monomers are extracted and with equilibration in WFI water for 92 hours, changing the water several times, typically 8 times during the 92 hours;
7. The purified gels are homogenised by grinding with a vertically oscillating grid;
8. A syringe is filled with the homogenised gel material;
9. Autoclavation of the syringe

A typical method for preparing the hydrogel may be summarised as:

### Preparation 1.2

### Process summary:

The gel is prepared by mixing an aqueous monomer solution of acrylamide (AM) and N,N'-methylene-bis-acrylamide (bisAM) as cross-linker with N,N,N',N'-tetramethylene ethylene diamine (TEMED) as co-initiator and ammonium persulfate (AMPS) as free-radical initiator (redox-system). By degassing a bulk solution with nitrogen, polymerisation starts. After final polymerisation the gel is transferred into a washing tank with net trays onto which the gel is placed. During water washing, the gel swells and monomer residues are extracted. The swollen gel is fed and evacuated in a filling unit having the gel delivered in a syringe, which is autoclaved.

Two alternate formulations have been prepared, a lower- and a higher- end elasticity formulation.

**Table 1**

| Chemical constituent | lower end elasticity | higher end elasticity |
|---|---|---|
| acrylamide | 502 g | 547 g |
| N,N'-methylene-bis-acrylamide | 2,2 g | 4,6 g |
| TEMED | 3,0 g | 2,6 g |
| AMPS | 5,4 g | 5,0 g |
| Non-pyrogenic water | Add 10 litre | Add 10 litre |

The above are typical preparations of the hydrogel and may be adjusted within certain ranges.

### Preparation 1.3

### Polyacrylamide formulations from inline cross-linking process

A particularly interesting method of preparing the hydrogels of the invention involves an inline cross-linking process. Two individual and eventually degassed flows, one being a pre-mix of acrylamide, methylene-bis-acrylamide (the cross-linker) and TEMED, the other being the AMPS initiator solution, are pumped into a static mixer for mixing, chemical initiation and subsequent extrusion downstream into a pipe reactor made of Teflon or steel in which the polymerisation occurs. Washing of the gel is simplified due to high surface area of gel from reactor.

By selecting monomer, cross-linker and initiator concentrations and their relative molar ratios, and by regulating the two flow rates and the polymerisation temperatures, it is possible to produce gels that are varying in degree of crosslinking and in solids content.

### Preparation 1.4

The reagents were combined in ratios described in Tables 2, 3 and 4, and washed as described in the Tables (with pyrogen-free water unless indicated otherwise) to give low, medium, and high elasticity formulations. Hydrogels with solid weight contents between 0.5 and 25% polyacrylamide were prepared.

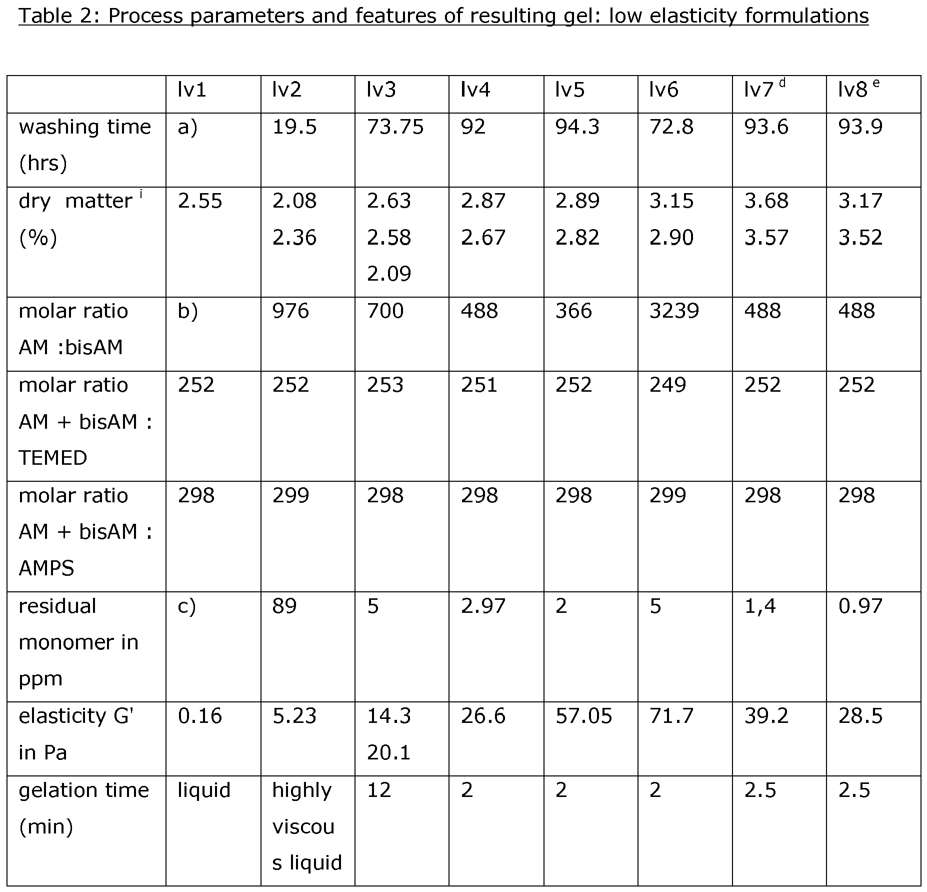

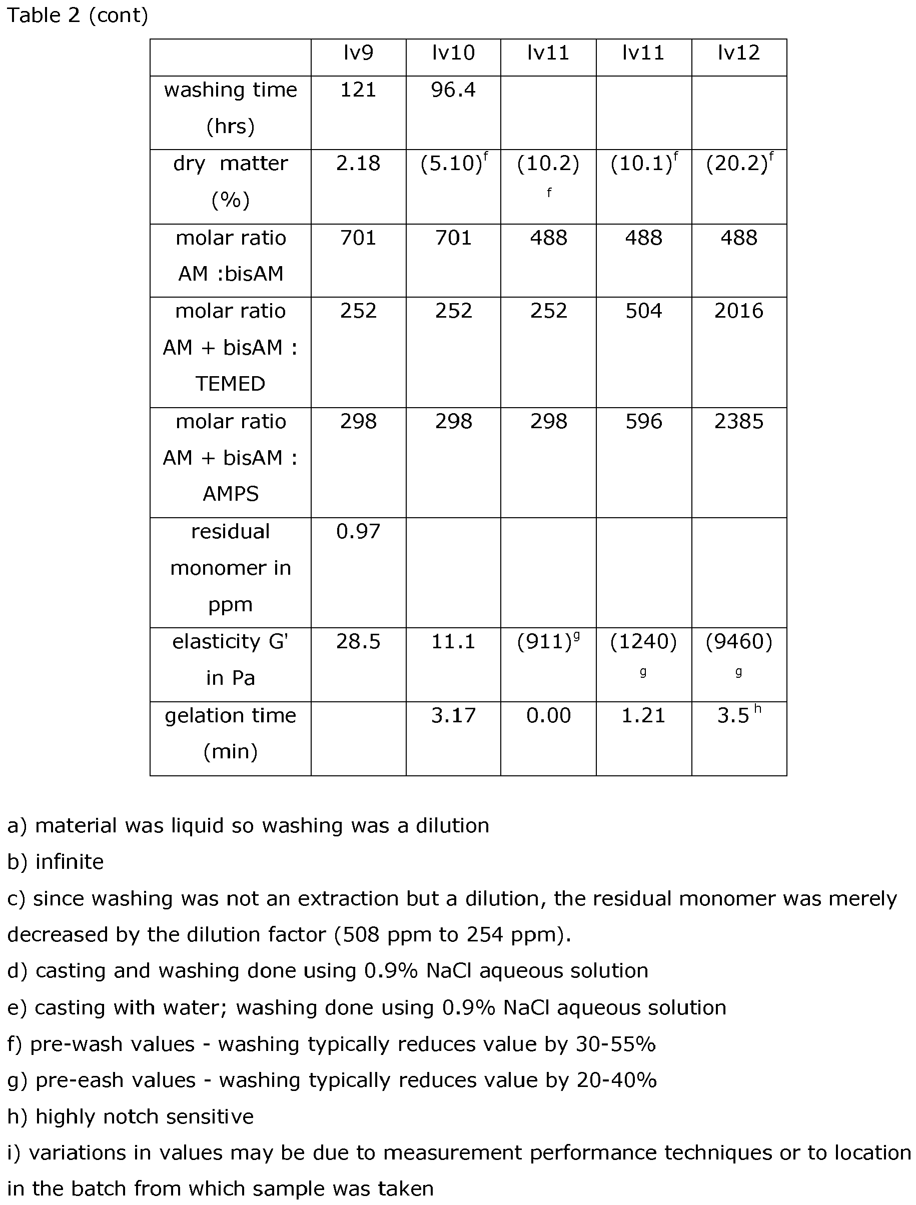

**Table 3: Process parameters and features of resulting gel: medium elasticity formulations**

| | mv1 | mv2 | mv3 | mv4 | mv5 |
|---|---|---|---|---|---|
| washing time (hrs) | 97 | 211.5 | 96 | 94.8 | 90.3 |
| dry matter (%) | 3.14 | 2.49 | 3.25 | 3.29 | 3.22 |
| molar ratio AM :bisAM | 310 | 310 | 290 | 289 | 289 |
| molar ratio AM + BISAM : TEMED | 252 | 252 | 252 | 251 | 252 |
| molar ratio AM + BISAM : APS | 299 | 299 | 299 | 299 | 299 |
| residual monomer in ppm | 1.6 | | 1.5 | | |
| elasticity G' in Pa | 108.5 | | 129 | 133.5 | |
| gelation time (min) | 2.5 | 2.5 | 2.18 | | |

**Table 4: Process parameters and features of resulting gel: high elasticity formulations**

| | hv1 | hv2 | hv3 | hv4 | hv5 |
|---|---|---|---|---|---|
| washing time (hrs) | 119.5 | 516 | 122 | 95.5 | 116.7 |
| dry matter (%) | 3.47 | 2.5 | 3.56 | 3.83 | 3.42 |
| molar ratio AM :bisAM | 260 | 260 | 260 | 260 | 260 |
| molar ratio AM + bisAM : TEMED | 315 | 315 | 604 | 313 | 314 |
| molar ratio AM + bisAM : AM PS | 376 | 376 | 755 | 375 | 376 |
| residual monomer in ppm | 0.2 | | | | |
| elasticity G' in Pa | 343 | 274 | | 314.5 | |
| gelation time (min) | 2.18 | 2.18 | 7.5 | | |

### Example 2

### PAAG gel tissue interaction in joints

### A pilot study of 4 rabbit knee and shoulder joints

### Clinics

A total of 4 rabbits (white New Zealand rabbits) were injected intraarticularly with PAAG on one side. The contra-lateral side was used as a control. Injection of PAAG was preceded by sucking out the same amount of joint fluid in order to keep a stable joint pressure.

The rabbits were kept under normal conditions, 2 and 2, for 10 days and were then sacrificed. All animals had behaved normally during the 10 days with no pain or discomfort.

### Macroscopy

At autopsy day 10 it was clear that not all PAAG had been injected intraarticularly, as some was identified in the loose connective or fat tissue surrounding the joints.

The gel was identified intraarticularly in all knee joints injected. It could not be identified macroscopically within the shoulder joints.

The contralateral side was completely normal-looking. All peri- and intraarticular tissues from injected and non-injected contra-lateral joints (mainly fat tissue and loose connective tissue) were examined histologically.

### Microscopy

The synovial lining of the joints was in all injected joints significantly enlarged due to a massive gel-uptake. A minimal amount of gel was seen corresponding to the joint cavity. Most of the gel was seen in intimate combination with the synovial lining cells of the interior of the joint capsule, forming a thick rim of macrophages (synovial cells) and gel. Most of the gel was seen between rather than within the phagocytic cells. Giant cells were very rare.

Deposits of gel were not seen, neither in accidentally injected fat tissue nor in loose connective tissue or muscle tissue.

The corresponding samples from the non-injected contra-lateral joints were all normal-looking.

### Conclusion

When injected into joints PAAG tends to enter the synovial lining of the inner capsule forming a thick membrane, which at 10 days consists of gel and macrophages. Further studies with longer observation times are needed in order to follow the fate and possible curative effect of PAAG on arthritic joints.

### Example 3

### Histology following injection with polyacrylamide hydrogel for visco-supplementation of the knee joint

A longitudinal study of soft tissue reactions to polyacrylamide hydrogel (PAAG)) and hyaluronic acid (Durolane®) in the rabbit

### Background:

Osteoarthritis is an increasingly common chronic joint disorder worldwide, which due to pain inactivates a large number of predominantly elderly or overweight persons. Previous studies on injection of hyaluronic acid gel (HAG) into the joint cavity have shown some pain relief and improved mobility with prolonged effects as compared to corticosteroids, but the effect is restricted to the 5 to 13 week post injection period, and repeated injections have been necessary.

Polyacrylamide hydrogel (PAAG) is, like HAG, a hydrophilic polymer gel with 97.5 % water, but unlike HAG this gel is non-degradable, as it consists of a stable, non-degradable, backbone of firmly bound, polyacrylamide, to which the many exchangeable water molecules are attached.

The purpose of this study was to investigate the effect on joint tissue of PAAG as compared to HAG, and follow the fate of the 2 gel types over time.

The brand name of HAG, which was used, is Durolane®.

### Materials and methods:

A total of 10 New Zealand white rabbits were used. The knee joints were injected. The first 4 rabbits were injected with PAAG on one the right side and saline (as control) on the left. The other 6 rabbits were injected with PAAG on the right side and HAG (as control) on the left.

The amount of injected gel was 0.3 ml, and the procedure was done after having removed a similar amount of joint fluid.

The rabbits were observed daily for well-being and possible physical or eating/drinking disorders and weight was controlled once a week.

The first 4 rabbits, which had been injected with PAAG on one side and saline on the other, were sacrificed after 10 days. The other 6 rabbits, which had been injected with PAAG on one and HAG on the other, were sacrificed, 2 at a time, at 3, 6 and 12 months.

For all rabbits both knee joints were removed and subjected to pathological investigation.

The knee joints were inspected macroscopically, and all joint synovial, tendinous or fat tissue was removed for histological preparation and examination. The tissues were stained with H&E and Van Gieson/Alcian blue.

### Results

Day 10: For all 4 rabbits the findings were the same. On the PAAG injected side the synovial lining was 5-10 fold thicker than on the other side, and PAAG was seen in the joint cavity as a homogenous mass or as thin threads. PAAG was also seen as an integrated part of the synovial lining, but the gel tended to lie around the synoviocytes (arrows) rather than within these. The synovial lining on the other side, which had been injected with saline, was normal-looking.

Day 90: The synovialis did not contain any PAAG, but the gel could be seen in the crevices between the folding membranes. HAG, in contrast, was seen abundantly within the synovial lining but not in the joint cavity.

PAAG was also seen in the loose connective or fat tissue, most likely a result of iatrogenic injection, due to technical difficulty in gaining needle access to the joint cavity only.

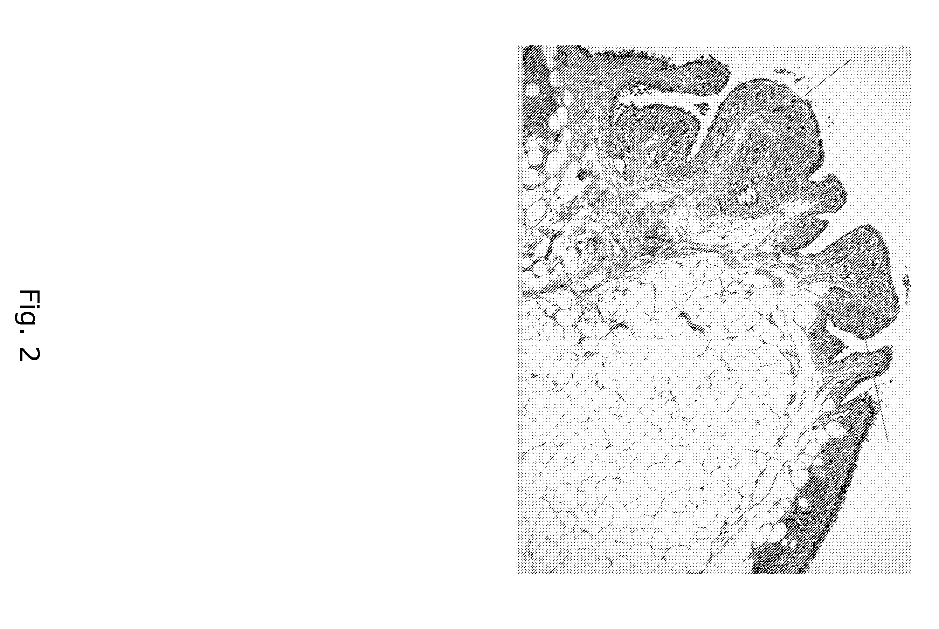

Day 180: Traces of PAAG remained in the crevices between synovialis-lined membranes and within adjacent loose connective tissue (Fig.2). Lining epithelium was unreactive. The synovial membranes remained thickened due to HAG infiltration, and on one occasion the gel was seen in the cavity.

Day 360: The same PAAG pattern was seen as at day 180, but PAAG was also seen in adjacent connective tissue and in connective tissue lying further away from the synovialis. In the latter 2 cases a typical vessel-bearing fibrous network was seen, HAG appeared to take up less space in the synovial lining, and the gel had shifted position from being around and inside the synoviocytes to be below these cells or even further away - in the connective tissue, where it appeared to be under degradation.

### Conclusion:

During the early stages of gel uptake (within 10 days) PAAG is intimately mixed with but not engulfed by the macrophage-like synoviocytes of the joint cavity. Later (at 3 months) the cells of the synovial membrane have returned to normal with no gel association other than gel still present in the joint cavity (both at 6 and 12 months).HAG (Durolane®) appeared to enter the synovial lining, at first as a thickening of the cells, having engulfed the gel, and later as a subepitelial rim. The gel was only seen once (at 6 months) within the joint cavity.

### Example 4

### Clinical evaluation of treatment of osteoarthritis in veterinary medicine - a report of treatment of 23 horses having joint arthritis with polyacrylamide hydrogel

This study describes clinical and histo-pathological findings in 23 horses originating from 3 different centers. Most of the horses had been injected intraarticularly with several different symptomatic substances before they were treated with PAAG in a final attempt to alleviate joint osteoarthritis and cure lameness.

### Normal procedure to diagnose joint arthritis

In order to identify which joint is suffering a local anaesthetic (i.e. lidocain) is injected into the cavity. This sedation allows free handling and bending of an otherwise stiff joint within 5 min.

### Initial treatment

The first choice in treating arthritis is typically the injection of a combination of steroid for anti-inflammatory effect and hyaluronic acid gel for visco-supplementation. A 14-days observation period follows, after which the effect should have set in. If not, the procedure is typically repeated 2-3 times before another option is tested, see below.

The second typical step will often be the autologous blood treatment by Interleukin-1 Receptor Antagonist Protein (IRAP), - a method based on re-injections of samples of the horse's own blood having been enriched with an increased quantity of anti-inflammatory proteins (IRAP). Blood re-injections take place over 1 year.

Another second step may be injection with Adequan, a polysulfated glycosaminoglycan liquid, which is injected directly into the joint cavity or i.m.

### Final treatment

The third and final step included in this study was injection with PAAG (Arthramid® Vet). The coffin joint, the fetlock joint and the knee joint of the hind legs, were the only joints treated. For antisepsis the leg hair covering the injection area was cropped and the skin thoroughly rinsed with chlorhexidine and ethanol (3 times interchangeably). The injection procedure is as follows:

The cannula (gauge 19) was inserted inside the joint cavity, and without extracting gel liquid PAAG (1-2 ml) was injected together with Gentamycin. Sterile gauze and a firm bandage was applied and kept on the leg for 24 h, and afterwards, the horse was kept in a box to rest for 5 days - only being taken out for a walk by hand for 10 min a day.

### Clinical results (Table 5)

The effect was not instantaneous but set in after a minimum of 4 days and a maximum of 6 weeks. If 2 ml of gel was injected, the total effect could already be seen at 1-2 weeks, whereas it did not set in until after 4-6 weeks, if only 1 ml was given. One of the first horses being injected with PAAG, showed a long-term effect of the injection, before it had to be put down due to causes not related to the gel injections (Table 5). The effect was still present at 8 months for fetlock and coffin joints and at 4½ months for knee joints.

Twenty-three horses had had been injected with the PAAG in a total of 36 joints, 12 coffin, 16 fetlock and 8 knee joints. The injections were carried out during little over one year, from 02.01-2009 to 10.03-2010, and because of the short follow-up for some horses, results were sparse. However, the majority of horses (n=16), (69%) stopped limping, and in most cases this lasted until follow-up (Table 5) or death from not-related causes (n=6, (26 %)). Only a minority of horses failed to show any improvement after the injection (n=5), and only 6 injected joints failed to show any effect (17%) - one with only 14 days and the other with just 1-month follow-up. Two joints had been injected so recently (within 14 days) that an effect was not expected to and had not set in yet. One knee joint had recurring symptoms after only 2 months, and one knee joint out of 5 from the same horse (2 coffin and 2 fetlock) only showed partial effect at 8 months. One running horse recovered completely and is doing well in competitions, now 7 months later, and another recovered so well, that it has come in first in 3 successive runs, now 5 months after the gel injection.

A total of 6 horses had to be put down due to reason unrelated to the gel injections. Only 1 horse was given up due to very severe osteoarthritis symptoms, which recurred only 2 months after the injection (knee joint).

Three of the 6 horses mentioned above were further examined post-mortem. The injected leg joints from these horses were studied macroscopically through dissection and microscopically by taking out relevant tissue samples from the gel-injected sites. The 3 horses had had the gel injected 14 days, 1 month and 8 months previously.

### Histopathological results

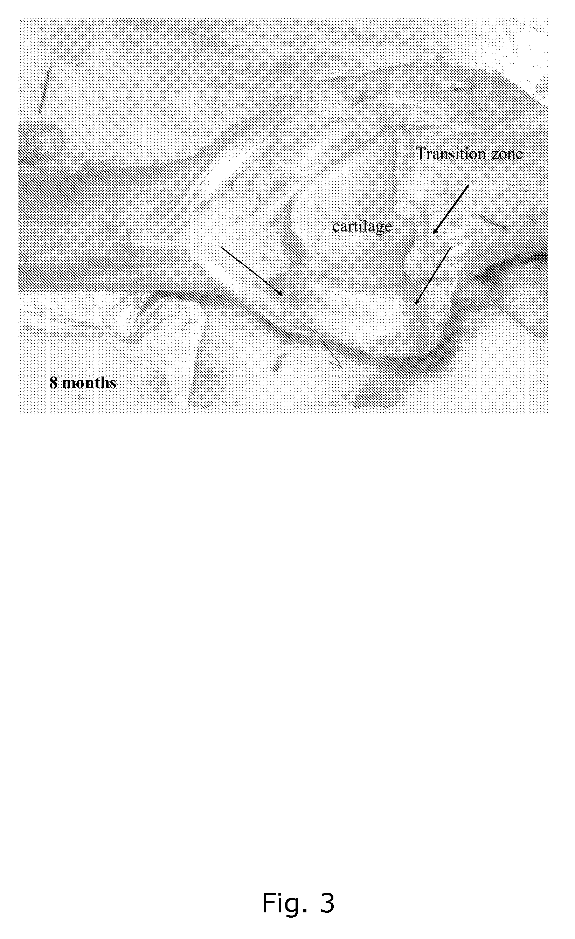

Macroscopically, the gel appeared as a thick slimy membrane within the joint cavity or as small clear deposits lying within the herniated fat tissue inside the joint. Free gel inside the cavity was only seen during the early phases (i.e. within 14 days after injection). One knee joint, which had harbored the gel for 8 months, showed a thick yellow lining of the inner side. The colour was a result of admixed blood pigment, as shown below. In a fetlock joint gel was found at the transition sites from connective tissue to cartilage (Fig. 3).

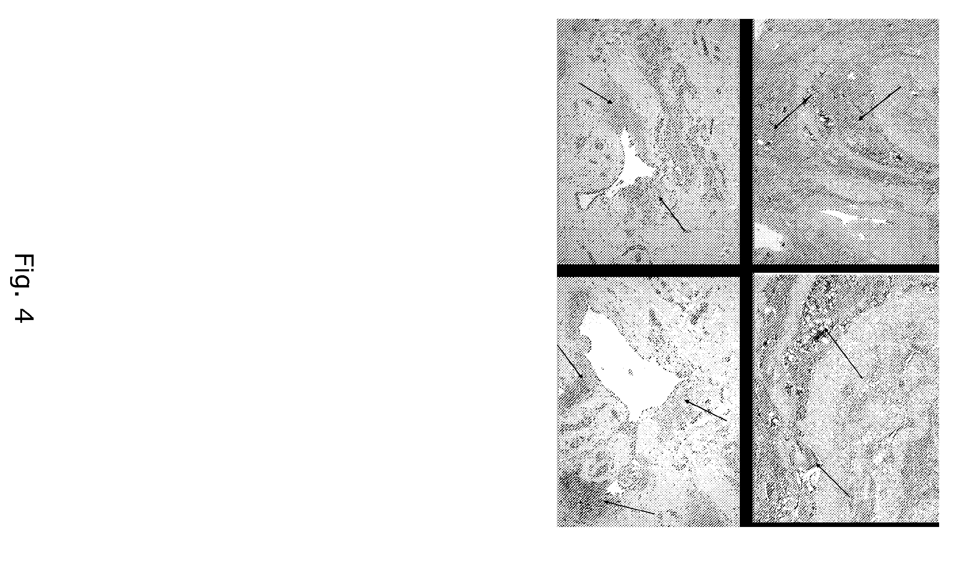

Microscopically, gel was seen as small deposits within the cavity and *among* the synovial cells at 14 days. At 1 month the gel appeared as membranes or cushions *below* these cells, and at 8 months gel was equally present lying within the connective tissue and the herniating fat below the cells of the inner synovial membrane (Fig. 4). The synovial cells contained blood pigment, giving the lining a yellow tinge macroscopically.

### Conclusion

This study has shown that injection of PAAG, preferably 2 ml per dose, into horse joints (coffin, fetlock and knee) suffering from osteoarthritis has a long-lasting effect, which prevents lameness. The mode of action appears to be through a buffer-like capacity of the synovial lining, as the gel becomes intimately integrated within the soft tissues herniating into the joint cavity. The gel is not being degraded, and it is possible that future, high-volume injections of this gel may exert it promising cushioning effect on the joint by not only supporting and preserving the synovial lining but perhaps also by allowing regeneration of the underlying damaged joint cartilage and soft tissues.

**Table 5.**

| Overview of 23 horses from 3 centers having been injected into a total of 36 osteoarthritic joints during a 1-year period. Three were examined post-mortem ( ). | | | | |
|---|---|---|---|---|
| **No** | **Joint** | **Amount** | **Success** | **Follow-up/death** |
| 1 | fetlock (2) | | | |
| | knee | 2 ml/joint | Yes | 4½ months † |
| | Knee | - | Yes | 3½ months |
| (3) | fetlock (2) | - | yes | |
| | coffin (2) | | yes | 8 months † |
| | knee | | partial | |
| (4) | coffin (2) | - | No | 14 days † |
| (5) | coffin (2) | 4 ml/joint | Yes, one side | 1 month † |
| 6 | coffin | 2 ml/joint | No | 2½ month † |
| 7 | fetlock (2) | 2 ml/joint | Yes | 7 months |
| 8 | coffin | 2 ml | Yes | 4½ months |
| 9 | coffin (2) | 2 ml/joint | Yes | 4½ month |
| 10 | knee (2) | - | only 2 months | 4 months |
| 11 | Knee | 2 ml/chamber | No | 4 months |
| 12 | fetlock (2) | 2 ml/joint | Yes | 5 months |
| 13 | fetlock | 1 ml | Yes | 5 months |
| 14 | Coffin | 1 ml + 2ml | Yes | 4 months |
| 15 | Fetlock | 1 ml | No | 5/2 months |
| 16 | Fetlock | - | Yes | 2 months † |
| 17 | Knee | 2 ml | No | 4 months |
| 18 | fetlock (2) | 1 ml/joint | Yes | 3 months |
| 19 | Fetlock | 1 ml | Yes | 1 month |
| 20 | fetlock (2) | 1 ml/joint | ? | ? |
| 21 | Fetlock | 1 ml | Yes | 5 months |
| 22 | Coffin | 1 ml | Yes | 5 months |
| 23 | Knee | 1 ml | ? | 14 days |

### Example 5

### Arthramid^{®} Vet polyacrylamide hydrogel (PAAG)

Osteoarthritis (OA) is a frequent cause of lameness in horses. Arthramid^{®} Vet for intra articular injection in horses suffering from OA provides long lasting relief of symptoms such as pain and lameness.

A minimum of 2 ml Arthramid^{®} Vet is injected into the joint cavity using an 18 - 21 gauge needle. Prophylactic antibiotic use is recommended.

The mode of action appears to be mechanical through a buffer-like capacity in the synovial lining, as the gel becomes intimately integrated within the soft tissues herniating into the joint cavity. The effect is seen after 2-3 weeks and is shown to last for at least 8 months.

After the Arthramid^{®} Vet injection, a gradually increasing training program is followed:
- 2 - 3 days of box rest
- first. week: 10 min. walk by hand daily
- second week: 10 min. walk by hand 2 twice daily
- third and fourth week: 30 min. of light exercise with rider daily

### Case story I - A 13 year old show jumper (no 7 in the above table)

A 13 year old show jumper - (140 / 145 cm level) was losing power and scope at shows. At first examination in April 2009 the horse showed 1/5 on the lameness scale and massive effusion in both front fetlocks.

### Clinical investigation

Flexion test gave a positive reaction in both front feet.

When injected with IA analgesia both front fetlocks blocked positive.

### Radiographic examination

X-ray showed osteoarthrosis in both front fetlocks.

### Treatment

The horse was treated with triamcinolone and hyaluronic acid 3 times, several times with IRAP, and the affected joints were flushed with saline. The horse responded moderate to well to these treatments, but lameness returned when resuming sport.

In September 2009 the horse was treated with Arthramid^{®} Vet gel, 2 ml in each joint.

### Outcome

At 2 weeks follow up, the horse was sound, and the flexion test was less positive. Sport was resumed, and at the 2-month follow up, the horse was still sound, now with negative flexion test.

At the 1-year follow up in September 2010 it was still lame free and competing at the same level as before. There was a normal amount of joint fluid in both fetlocks.

### Case story II - 12 years old show jumper

12 years old show jumper was examined in October 2010. The horse became lame after a jumping accident, and the lameness had persisted for 1-year.

### Clinical examination

The right hind leg showed 3/5 on the lameness scale on both hard and soft ground. The flexion test was positive.

### Radiographic examination

The radiograms (anterior-posterior projection) showed a fissure of the dorsal area of the proximal phalanx of the right hind leg and periosteal reactions of the proximal phalanx.

### MRI

MRI confirmed the fissure of the dorsal area of the proximal phalanx, accumulation of fluid in the area and normal cartilage.

### Treatment

The horse was injected intraarticularly with 2 ml of Arthramid Vet^{®} into the fetlock joint of the right hind leg. In addition, an intravenous infusion of tiludronate (bisphosphonate) was administered.

After a rest for 5 days the horse started walking for about 15 minutes a day.

### Outcome

At 1-month follow up the horse was sound on both hard and soft ground.

Training was slowly initiated, and 2 months after injection the horse was still sound. The owners of the horse are very satisfied with the outcome.

### Case story III - a 14-year old show-jumping horse

A 14-year old show-jumping horse was presented to an equine clinic. It had been lame on the left front leg for about 5 months. Six weeks ago it had been suffering from an iatrogenic septic inflammation of the fetlock joint of the left front. The joint was treated by repeated intraarticular joint lavages and local systemic antibiotics.The grade of lameness improved, but the horse never returned sound.Local treatments consisting of injections of corticosteroids and hyaluronic acid were performed, but did not improve the grade of lameness.

### Clinical examination

The clinical examination was performed in walk and trot on hard as well as on soft ground. The horse showed a mild to moderate lameness of the left front leg ( 2 / 5 ).

The anesthesia of the fetlock joint was positiv.

### Radiographic examination

The radiographs showed a marked osteoarthritis of the fetlock joint. Furthermore a subchondral cyst in the distal part of the third metacarpal bone was clearly visible.

### Scintigraphy

A scintigraphy was performed. One hot spot was found in the area of the fetlock joint of the left front leg.

### Treatment

A 2 ml injection of ArthramidVet® into the fetlock joint of the left front leg was performed.

### Outcome

After the treatment the horse had rest for 5 days and afterwards started walking again. The follow up examination after one month showed that the horse was free of lameness and training was started again.

### Case story IV - A 10-year old show-jumping horse

A 10-year old show-jumping horse was presented to an equine clinic showing lameness of the right front in varying intensities for about two years. The radiographic examination displayed mild osteoarthritis/DJD of the coffin joint with a irregular horizontal axis A MRI was performed that revealed moderate cartilage damage in the coffin joint of the right front foot.

As treatment, the horse started to receive injections of cortocosteroids plus hyaluronic acid. After two days of rest and 2 weeks of walk the training was picked up but the lameness reoccurred. Consequently 3 interval injections of IRAP® with 14 days apart into the coffin joint were performed but every time the training was resumed to higher levels the lameness reoccured.

### Clinical examination

The lameness examination was performed in walk and trot on hard and soft ground.

The horse showed a moderate ( 3 / 5 ) lameness of the right front leg on hard as well as on soft ground.The coffin joint block of the right front leg was completely positive without shift to the other leg.

### Radiographic examination

The radiographs showed mild signs of DJD of the coffin joint of the right front.

### MRI

The MRI showed mild cartilage damage in the coffin joint of the right front.

### Treatment

The horse was finally treated by a local 2 ml injection of ArthramidVet® into the coffin joint of the right front leg. After the treatment it had box rest for 2 days and started a walking regime for another 12 days.

### Outcome

One month later a follow up examination was performed. The horse was free of lameness on soft as well as on hard ground. Consequently training was picked and the horse entered competition level since then.

### Example 6

### Treatment of cats and dogs

A cat has been treated with injection of 2 x 0.5 hydrogel in both knees in May 2008. At a follow up after 2 months the cat's symptoms had improved significantly.

This case story inspired to the study of the effect in dogs which is described in the table below.

**Table 6.**

| Dogs suffering from osteoarthritis injected intraarticularly with Arthramid® Vet in chronological order | | | | | | | |
|---|---|---|---|---|---|---|---|
| **Gender, name, age** | **Breed** | **Affected Joints** | **Date 1st treatment** | **Date 2nd treatment** | **Volume (ml)** | **Success after?** | **Other treatment before/after** |
| M. Barney, 3 years 4 months | Labrador retriever | Osteochondrosis Both front elbows | 11.10.10 | | 0,7 ml per joint | Yes, 16.11.10 | Before: Cartrophen, Rimadyl, glucosamine Chondroitin etc. |
| F.Freja, 7 years 3 months | Rottweiler | Right front elbow | 29.10.10 | | 0,7 ml | No, 16.11.10 lame again | ? |
| F. Rosa 9 years 4 months | Mixed breed | Left back knee | 05.11.10 | | 0,5 ml | | Before: Rimadyl, Forthyron |
| M. Tjabo 10 years 1 month | Labrador retriever | Right front elbow | 11.11.10 | | 0,5 ml | Yes, 16.11.10 | Before: Cartrophen, |
| F. Lykke 10 years 1 month | German Shepherd | Right back knee | 11.11.10 | | 0,5 ml | | Before Cartrophen, Rimadyl, A-cell |
| F. S. Pitzner 7½ months | Labrador retriever | Right front elbow | | | 0,4 ml | No, lame again | |
| M. Cato 3 years | Labrador retriever | Both front elbows | 16.08.10 | | 0,4 ml | Yes, 13.09.10 Free of lameness | Before diet, Compartin, arthroscopy with removal of joint mouse |
| F. (Hazel) 8 months | Labrador | Both front elbows | | | 0,4 ml | Better, but some ROM, no medicine | Compartin? |
| M. Chico 1 year | Rottweiler | Elbow, both? | 22.06.10 | | 0,5 ml | 06.07.10 better | ? |
| M Bailey 2 years 2 months | Labrador Retriever | Both front elbows | 08.12.09 | | 0,4 ml | ? | Like Cato |

### Example 7

### Treatment of humans

Results of treatment of humans with PAAG are summarized in the table below.

**Table 7**

| **Initials and age** | **Right knee or hip** | **Left knee or hip** | **Status** | **Comments** |
|---|---|---|---|---|
| FH, 56 years | 29.01.10, 1 ml | 29.01.10, 1 ml | Swelling reduced | Is presently operated 2 times in left knee and 3 times in right knee |
| | 22.03.10, 1 ml | 22.03.10, 1 ml | | |
| | | 04.05.10, 2 ml | | |
| EVN, 64 years | 18.05.10, 2 ml | 18.05.10, 2 ml | Able to play 9 hole golf 2 times a week and does not use painkillers anymore | It was the best what happened in the patient's life. Cancelled knee replacement surgery. |
| | | 17.11.10, 1 ml | | |
| IMJ, 68 years | 15.04.10, 2 ml | | Clear improvement and full ROM (range of movement) 14.07.10 | Discontinued use of pain killers |
| | 03.06.10, 2 ml | | | |
| OHN, 69 years | 14.06.10, 2 ml | 13.04.10, 2 ml | Swelling reduced in both knees | Received new knee |
| | | 14.06.10, 2 ml | | |
| PMH, 72 years | 11.02.10, 2 ml | | Significant improvement | |
| | 22.04.10, 2 ml | | | |
| MIT, 81 years | 08.03.10, 2 ml | | 07.06.10 very | |
| | 07.06.10, 1 ml | | satisfying result | |
| TF, 63 years | 26.04.10, 2 ml | | Swelling reduced | |
| | 11.05.10, 2 ml | | | |
| ML, 61 years | 04.05.10, 2 ml | | | |
| | in left hip | | | |
| SV, 52 years | 24.06.10, 2 ml | | 01.10.10 right side good result, left side moderate pain | |
| | in both hip joints | | | |
| | 18.08.10, 2 ml | | | |
| | in both hip joints | | | |

### Case story 1 - Male, 56 years old, bilateral knee OA

### Male, FH 56 years old, bilateral knee OA

- Pain under physical load
- Repeated swelling, emptied up to 40 ml liquid several times
- Enjoyes playing tennis, but plays very seldom due to strong pain
- Orthopedic surgeons evaluate that alloplasty will be necessary within next 5 years
- 29.01.2010 1 ml PAAG in each knee
- 22.03.2010 1 ml PAAG in each knee
- 16.04.2010 Patient is cycling, swimming and playing tennis
- every second day, both knees are dry

### Case story 2- Male, bilateral knee osteoarthrosis

### Male, PMH , bilateral knee osteoarthrosis

■ 11.02.2010 2 ml PAAG in total
■ 16.04.2010 Significantly improved in 6 weeks, heavy work with horses and construction, some worsening, but still much better as before injection

### Case story 3 -Female, 80 years referred to total alloplasty of the right knee

Female, MIT, 80 years old, referred to total alloplasty of the right knee
■ Max walking distance 600 m
■ Strong pain upon movement
■ Massive pathological changes in the joint
■ 08.03.20010 2 ml PAAG in the right knee
■ 16.04.2010 Significant improvement, does not use supporting bandage, easily walks stairs in the morning

### REFERENCE LIST

Liu YZ, Jackson AP, Cosgrove SD. Contribution of calcium-containing crystals to cartilage degradation and synovial inflammation in osteoarthritis. Osteoarthritis and Cartilage 17, 1333-1340, 2009.

## Claims

1. A hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution for use in the treatment and/or prevention of joint swelling and/or bone oedema in a mammal suffering from arthritis.

2. A hydrogel according to claim 1 wherein the hydrogel consists of 97.5% apyrogenic water and 2.5% polyacrylamide.

3. A hydrogel according to claim 1 or 2 wherein the administration of the hydrogel is performed by intraarticular injection under sterile conditions.

4. A hydrogel according to claim 3 wherein 0.25 ml to 10 ml is to be injected.

5. A hydrogel according to any one of claims 1-4 wherein the mammal suffering from arthritis is a human, a racing animal or a companion animal.

6. A hydrogel according to any one of claims 1-5 wherein the mammal to be treated is a human and the joint or joints which is/are to be injected is the knee, hip, elbow, the metacarpal-phalangeal and interphalangeal joints in hands and feet, the sesamoid joint and/or the temperomandibular joint.

7. A hydrogel according to any one of claims 1-5 wherein the mammal to be treated is a horse and the joint or joints which is/are to be injected is the fetlock, coffin, pastern, stifle, and/or knee joint of the hind legs.

8. A hydrogel according to any one of claims 1-5 wherein the mammal to be treated is a dog and the joint or joints which is/are to be injected is the elbow of the front leg or the knee or hip joint of the hind legs.

9. A hydrogel according to any one of claims 1-8 wherein the mammal is suffering from osteoarthritis.

10. A method of treating and/or preventing joint swelling and/or bone oedema in a mammal suffering from arthritis, the method comprising injecting intraarticularly under sterile conditions a hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution.

11. A method according to claim 10 wherein the hydrogel consists of 97.5% apyrogenic water and 2.5% polyacrylamide.

12. A method according to claim 10 wherein 0.25 ml to 10 ml is injected.

13. A method according to claim 10 wherein the mammal suffering from arthritis is a human, a racing animal or a companion animal.

14. A method according to claim 10 wherein the mammal is suffering from osteoarthrosis.
